# EUROPÄISCHE PATENTSCHRIFT

(11) **EP 0 953 347 B1**
(45) Veröffentlichungstag und Bekanntmachung des Hinweises auf die Patenterteilung: **13.02.2008**
(21) Anmeldenummer: 99106025.2
(22) Anmeldetag: 25.03.1999
(51) Int. Cl.: A61K 9/16, A61K 9/20, A61K 9/48, A61K 47/34

(54) **Verwendung von Polyalkylenoxid-haltigen Pfropfpolymerisaten als Solubilisatoren**
Use of graft polymers containing polyalkyleneoxides as solubilizers
Utilisation de polymères greffés d'oxyde de polyalkylène en tant qu'agents solubilisants

(30) Priorität: 02.04.1998 DE 19814739
(43) Veröffentlichungstag der Anmeldung: 03.11.1999
(73) Patentinhaber: BASF Aktiengesellschaft, 67056 Ludwigshafen (DE)
(72) Erfinder: Huff, Jürgen, Dr., 67063 Ludwigshafen (DE); Meffert, Helmut, Dr., 68161 Mannheim (DE); Ruchatz, Folker, Dr., 67433 Neustadt (DE)

(56) Entgegenhaltungen:
- EP-A- 0 219 048
- EP-A- 0 285 935
- EP-A- 0 950 404
- GB-A- 922 457

## Beschreibung

Die Erfindung betrifft die Verwendung von Polyalkylenoxid-haltigen Pfropfpolymerisaten als Solubilisatoren.

Bei der Herstellung homogener pharmazeutischer oder kosmetischer Zubereitungen hat die Solubilisierung von hydrophoben Stoffen eine sehr große praktische Bedeutung erlangt.

Unter Solubilisierung ist eine Löslichkeitsverbesserung durch amphiphile Verbindungen zu verstehen, die in der Lage sind, schlecht wasserlösliche oder wasserunlösliche Stoffe in klare, höchstens opaleszierende wäßrige Lösungen zu überführen, ohne daß hierbei die chemische Struktur dieser Stoffe eine Veränderung erfährt.

Die hergestellten Solubilisate sind dadurch gekennzeichnet, daß der schlecht wasserlösliche oder wasserunlösliche Stoff in den Molekülassoziaten der amphiphilen Verbindungen, die sich in wäßriger Lösung bilden - den sogenannten Mizellen - gelöst vorliegt. Die resultierenden Lösungen sind stabile einphasige Systeme, die optisch klar bis opaleszent erscheinen und ohne Energieeintrag hergestellt werden können.

Solubilisatoren können beispielsweise das Aussehen von kosmetischen Formulierungen sowie von Lebensmittelzubereitungen verbessern, indem sie die Formulierungen transparent machen. Außerdem kann im Falle von pharmazeutischen Zubereitungen auch die Bioverfügbarkeit und damit die Wirkung von Arzneistoffen durch die Verwendung von Solubilisatoren gesteigert werden.

Als Solubilisatoren für pharmazeutische Arzneistoffe und kosmetische Wirkstoffe werden hauptsächlich folgende Produkte eingesetzt:
- ethoxiliertes (hydriertes) Ricinusöl, (z.B. Cremophor^{®} Marken, Fa. BASF);
- ethoxilierte Sorbitanfettsäureester, (z.B. Tween^{®} Marken, Fa. ICI);
- ethoxilierte Hydroxystearinsäure, (z.B. Solutol^{®} HS 15, Fa. BASF).

Die oben beschriebenen, bisher eingesetzten Solubilisatoren zeigen jedoch eine Reihe anwendungstechnischer Nachteile.

So ist z. B. deren parenterale Applikation mit einer Freisetzung von Histamin und einem daraus resultierenden Blutdruckabfall verbunden (Lorenz et al., Agents and Actions, Vol. 12, 1/2, 1982).

Die bekannten Solubilisatoren besitzen für einige schwerlösliche Arzneistoffe wie z.B. Clotrimazol nur eine geringe lösungsvermittelnde Wirkung.

Oberflächenaktive Verbindungen besitzen häufig eine hohe hämolytische Aktivität, die einer Anwendung auf dem Gebiet der Pharmazie, insbesondere in Parenteralia entgegensteht

In der GB 922,457 A ist die Herstellung von Pfropfpolymerisaten aus Polyalkylenoxiden und Vinylestern oder Estern der Methacrylsäure oder der Acrylsäure beschrieben, weiterhin auch deren Verwendung in Kaugummi oder Haarbehandlungsmitteln.

EP-A-0 219 048 beschreibt die Verwendung von Pfropfpolymerisaten aus Polyalkylenoxiden und Vinylacetat als Vergrauungsinhibitoren beim Waschen und Nachbehandeln von Synthesefasern enthaltendem Textilgut.

Es bestand nun die Aufgabe, neue Solubilisatoren für pharmazeutische, kosmetische sowie lebenmitteltechnische Anwendungen bereitzustellen, die die oben genannten Nachteile nicht aufweisen.

Diese Aufgabe wurde gelöst durch die Verwendung von Pfropfpolymerisaten, die erhältlich sind durch Pfropfen von
a) Polyalkylenoxiden mit
b) mindestens einem Monomeren, ausgewählt aus der Gruppe
   b₁) C₁-C₃₀-Alkylester von monoethylenisch ungesättigten C₃-C₈-Carbonsäuren;
   b₂) Vinylester von aliphatischen C₁-C₃₀-Carbonsäuren;
   b₃) C₁-C₃₀-Alkyl-Vinylether;
   b₄) N-C₁-C₃₀-Alkyl-substituierter Amide von monoethylenisch ungesättigten C₃-C₈-Carbonsäuren;
   b₅) N,N-C₁-C₃₀-Dialkyl-substituierter Amide von monoethylenisch ungesättigten C₃-C₈-Carbonsäuren
als Solubilisator.

Als Pfropfgrundlage a) können generell Polyalkylenoxide, insbesondere solche mit einem mittleren Molekulargewicht von 300 bis 100.000 g/mol auf Basis von Ethylenoxid, Propylenoxid und/oder Butylenoxid verwendet werden.

Bevorzugt verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 Mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 Mol.-%, der Propylenoxidanteil 1 bis 60 Mol.-% und der Anteil an Butylenoxid in den Copolymerisaten 1 bis 30 Mol.-%. Neben geradkettigen können auch verzweigte Homo- oder Copolymerisate als Pfropfgrundlage verwendet werden.

Verzweigte Copolymerisate können hergestellt werden, indem man beispielsweise an mehrwertige niedrigmolekulare Alkohole, z.B. Trimethylolpropan, Pentosen oder Hexosen, Ethylenoxid und gegebenenfalls noch Propylenoxid und/oder Butylenoxide anlagert. Die Alkylenoxid-Einheiten können im Polymerisat statistisch verteilt sein oder in Form von Blöcken vorliegen.

Besonders bevorzugt verwendet man als Komponente a) Polyethylenoxide eines mittleren Molekulargewichts von 500 bis 20.000, insbesondere 800 bis 10.000 g/mol.

Es ist aber auch möglich, Polyester von Polyalkylenoxiden und aliphatischen oder aromatischen Dicarbonsäuren, z.B. Oxalsäure, Bernsteinsäure oder Adipinsäure mit Molmassen von 1500 bis 25.000, beschrieben in EP-A-0 743 962, als Pfropfgrundlage zu verwenden.

Für die Aufpfropfung auf die Polyalkylenoxide seien als Komponente b) folgende copolymerisierbare Monomere genannt:
C₁-C₃₀-Alkylester von monoethylenisch ungesättigten Carbonsäuren mit 3 bis 8 C-Atomen, wie z.B. Acrylsäure, Methacrylsäure, Dime-thacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure.

Als Alkoholkomponente der o.g. Ester seien C₁-C₃₀-Alkohole, bevorzugt C₁-C₁₂-Alkohole, besonders bevorzugt C₁-C₆-Alkohole genannt.

Aus der Gruppe der monoethylenisch ungesättigten Carbonsäuren werden bevorzugt Acrylsäure, Methacrylsäure, Maleinsäure oder Mischungen der genannten Carbonsäuren verwendet.

Besondere Bedeutung kommt hierbei den Acryl-, Methacryl- sowie Maleinsäureestern mit Alkoholen einer Kettenlänge von 1 bis 12, bevorzugt 1 bis 6 Kohlenstoffatomen zu.

Insbesondere seien hier genannt: Methylacrylat, Ethylacrylat, n-Propylacrylat, iso-Propylacrylat, n-Butylacrylat, 1-Methylpropylacrylat, 2-Methylpropylacrylat, n-Hexylacrylat, n-Heptylacrylat, n-Octylacrylat, 2-Ethylhexylacrylat, Nonylacrylat, Decylacrylat, Laurylacrylat, Methylmethacrylat, Ethylmethacrylat, n-Propylmethacrylat, iso-Propylmethacrylat, n-Butylmethacrylat, 1-Methylpropylmethacrylat, 2-Methylpropylmethacrylat, n-Hexylmethacrylat, n-Heptylmethacrylat, n-Octylmethacrylat, 2-Ethylhexylmethacrylat, Nonylmethacrylat, Decylmethacrylat, Laurylmethacrylat.

Als weitere Komponente b) können Vinylester aliphatischer, gesättigter oder ungesättigter C₁-C₃₀-Carbonsäuren, wie z.B. Ameisensäure, Essigsäure, Propionsäure, Buttersäure, Valeriansäure, Isovaleriansäure, Capronsäure, Caprylsäure, Caprinsäure, Undecylensäure, Laurinsäure, Myristinsäure, Palmitinsäure, Palmitoleinsäure, Stearinsäure, Ölsäure, Arachinsäure, Behensäure, Lignocerinsäure, Cerotinsäure sowie Melissensäure eingesetzt werden.

Bevorzugt werden Vinylester der oben genannten C₁-C₁₂-Carbonsäuren, insbesondere C₁-C₆-Carbonsäuren verwendet.

Ferner können C₁-C₃₀-Alkyl-Vinylether, bevorzugt C₁-C₁₂-Alkyl-Vinylethe=, besonders bevorzugt C₁-C₆-Alkylvinylether pfropfpolymerisiert werden.

Als bevorzugte Alkylreste der Vinylether seien verzweigte oder unverzweigte C₁-C₆-Alkylketten wie z.B. Methyl, Ethyl, n-Propyl, iso-Propyl, n-Butyl, 2-Methylpropyl, 1-Methylpropyl, n-Pentyl sowie n-Hexyl genannt.

Weiterhin können als Komponente b) N-C₁-C₃₀-Alkyl- oder N,N-C₁-C₃₀-Dialkyl-substituierte Amide von monoethylenisch ungesättigten C₃-C₈-Carbonsäuren verwendet werden, wobei es sich bei den Alkylresten um aliphatische oder cycloaliphatische Alkylreste mit 1 bis 30, bevorzugt 1 bis 12, besonders bevorzugt 1 bis 6 Kohlenstoffatomen handelt.

Unter den monoethylenisch ungesättigten Carbonsäuren mit 3 bis 8 C-Atomen sind ebenfalls die bereits oben genannten Säuren, bevorzugt Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure zu verstehen.

Aus dieser Gruppe von Monomeren werden ebenfalls besonders bevorzugt Acrylsäure, Methacrylsäure, Maleinsäure oder Mischungen der genannten Carbonsäuren verwendet.

Bevorzugte amidierte Comonomere sind beispielsweise N-Methylacrylamid, N-Methylmethacrylamid, N-Ethylacrylamid, N-Ethylmethacrylamid, N-n-Propylacrylamid, N-n-Propylmethacrylamid, N-iso-Propylacrylamid, N-iso-Propylmethacrylamid, N-n-Butylacrylamid, N-n-Butylmethacrylamid, N-1-Methylpropylacrylamid, N-1-Methylpropylmethacrylamid, N-2-Methylpropylacrylamid, N-2-Methylpropylmethacrylamid, N-n-Hexylacrylamid, N-n-Hexylmethacrylamid, N-n-Heptylacrylamid, N-n-Heptylmethacrylamid, N-n-Octylacrylamid, N-n-Octylmethacrylamid, N-2-Ethylhexylacrylamid, N-2-Ethylhexylmethacrylamid, N-Nonylacrylamid, N-Nonylmethacrylamid, N-Decylacrylamid, N-Decylmethacrylamid, N-Laurylacrylamid, N-Laurylmethacrylamid.

Selbstverständlich können auch Mischungen der jeweiligen Monomeren aus der Gruppe b) pfropfpolymerisiert werden.

Die hydrophoben Monomeren können daneben auch in Mischung mit einem oder mehreren hydrophilen Comonomeren eingesetzt werden. Verwendbar sind ethylenisch ungesättigte Carbonsäuren wie Acrylsäure, Methacrylsäure, Maleinsäure, Fumarsäure, Itaconsäure, Aconitsäure, daneben auch N-Vinylpyrrolidon, N-Vinylimidazol oder N-Vinylcaprolactam.

Die erfindungsgemäß verwendeten Pfropfpolymerisate haben ein mittleres Molekulargewicht von 1000 bis 30000 g/mol, bevorzugt 2000 bis 20000 g/mol, besonders bevorzugt 3000 bis 8000 g/mol.

Die Pfropfpolymerisate besitzen K-Werte von mindestens 7, vorzugsweise 10 bis 30, besonders bevorzugt 10 - 25. Die K-Werte werden bestimmt nach H. Fikentscher, Cellulose-Chemie, Band 13, 58 bis 64 und 71 bis 74 (1932) in Aceton bei 25°C und Polymerkonzentrationen, die je nach K-Wert-Bereich zwischen 0,1% und 5% liegen.

Zur Herstellung der Pfropfpolymerisate werden die in Betracht kommenden Polyalkylenoxide der Komponente (a) mit den Monomeren der Komponente (b) in Gegenwart von Radikale bildenden Initiatoren oder durch Einwirkung energiereicher Strahlung, worunter auch die Einwirkung energiereicher Elektronen verstanden werden soll, gepfropft.

Hierbei kann man so vorgehen, daß man das Polyalkylenoxid in mindestens einem Monomer der Gruppe (b) löst und nach Zugabe eines Polymerisationsinitiators die Mischung auspolymerisiert. Die Pfropfpolymerisation kann auch halbkontinuierlich durchgeführt werden, indem man zunächst einen Teil, z.B. 10 % des zu polymerisierenden Gemisches aus Polyalkylenoxid, mindestens einem Monomeren der Gruppe (b) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Pfropfpolymerisate können auch dadurch erhalten werden, daß man die Polyalkylenoxide der Gruppe (a) in einem Reaktor vorlegt, auf die Polymerisationstemperatur erwärmt und mindestens ein Monomer der Gruppe (b) und Polymerisationsinitiator entweder auf einmal, absatzweise oder vorzugsweise kontinuierlich zufügt und polymerisiert.

Das Gewichtsverhältnis der Komponenten (a) : (b) beträgt von 1 : 0,2 bis 1 : 10 und liegt vorzugsweise in dem Bereich von 1 : 0,5 bis 1 : 5.

Als Polymerisationsinitiatoren eignen sich vor allem organische Peroxide, wie Diacetylperoxid, Dibenzoylperoxid, Succinylperoxid, Di-tert.-butylperoxid, tert.-Butylperbenzoat, tert.-Butylperpivalat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid sowie Mischungen der genannten Initiatoren, Redoxinitiatoren und Azostarter.

Die verwendeten Mengen an Initiator bzw. Initiatorgemischen bezogen auf eingesetztes Monomer liegen zwischen 0,01 und 10 Gew.-%, vorzugsweise zwischen 0,3 und 5 Gew.-%.

Die Pfropfpolymerisation erfolgt im Temperaturbereich von 50 bis 200°C, bevorzugt im Bereich von 60 bis 140°C, besonders bevorzugt im Bereich von 70 bis 110°C. Sie wird üblicherweise unter atmosphärischem Druck durchgeführt, kann jedoch auch unter vermindertem oder erhöhtem Druck, vorzugsweise zwischen 1 und 5 bar ablaufen.

Falls gewünscht, kann die oben beschriebene Pfropfpolymerisation auch in einem Lösemittel durchgeführt werden. Geeignete Lösemittel sind beispielsweise Alkohole, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Die Pfropfpolymerisation kann auch in Wasser als Lösemittel durchgeführt werden. In diesem Fall liegt zunächst eine Lösung vor, die in Abhängigkeit von der Menge der zugegebenen Monomeren der Komponente b) in Wasser mehr oder weniger gut löslich ist. Um wasserunlösliche Produkte, die während der Polymerisation entstehen können in Lösung zu überführen, kann man beispielsweise organische Lösemittel zusetzen, wie einwertige Alkohole mit 1 bis 3 Kohlenstoffatomen, Aceton oder Dimethylformamid. Man kann jedoch auch bei der Pfropfpolymerisation in Wasser so verfahren, daß man die wasserunlöslichen Pfropfpolymerisate durch Zugabe üblicher Emulgatoren oder Schutzkolloide, z.B. Polyvinylalkohol, in eine feinteilige Dispersion überführt.

Als Emulgatoren verwendet man beispielsweise ionische oder nichtionische Tenside, deren HLB-Wert im Bereich von 3 bis 13 liegt. Zur Definition des HLB-Werts wird auf die Veröffentlichung von W.C. Griffin, J. Soc. Cosmetic Chem., Band 5, 249 (1954) hingewiesen.

Die Menge an Tensiden, bezogen auf das Pfropfcopolymerisat, beträgt 0,1 bis 5 Gew.%. Bei Verwendung von Wasser als Lösemittel erhält man Lösungen bzw. Dispersionen der Pfropfpolymerisate. Sofern man Lösungen des Pfropfpolymerisates in einem organischen Lösemittel herstellt bzw. in Mischungen aus einem organischen Lösemittel und Wasser, so verwendet man pro 100 Gew.-Teile des Pfropfpolymerisates 5 bis 200, vorzugsweise 10 bis 100 Gew.-Teile des organischen Lösemittels oder des Lösemittelgemisches.

### Anwendungen:

Durch die vorliegende Erfindung werden amphiphile Verbindungen für die Anwendung als Lösungsvermittler für pharmazeutische und kosmetische Zubereitungen sowie für Lebensmittelzubereitungen zur Verfügung gestellt. Sie besitzen die Eigenschaft, schwer lösliche Wirkstoffe auf dem Gebiet der Pharmazie und Kosmetik, schwerlösliche Nahrungsergänzungsmittel, beispielsweise Vitamine und Carotinoide aber auch schwerlösliche Wirkstoffe für den Einsatz in Pflanzenschutzmitteln sowie veterinärmedizinische Wirkstoffe zu solubilisieren.

Überraschend wurde bei den beanspruchten Verbindungen ein gutes Solubilisationsvermögen für pharmazeutische und kosmetische Wirkstoffe gefunden. Ferner werden mit den beanspruchten Verbindungen Anwendungen erhalten, die sich durch eine sehr geringe Hämolyserate, einer nebenwirkungsfreien Verträglichkeit nach parenteraler, oraler und topischer Applikation auf Haut und Schleimhaut auszeichnen. Die Verbindungen besitzen insbesondere keine Nebenwirkungen durch Wechselwirkungen mit Blutkörperchenmembranen. Nach parenteraler Applikation findet keine bzw. nur eine geringe Histaminfreisetzung statt. Die Solubilisatoren sind aufgrund ihres geringen Molekulargewichts nierengängig.

### Solubilisatoren für Kosmetik:

Die erfindungsgemäß verwendeten Pfropfpolymerisate können als Solubilisatoren in kosmetischen Formulierungen eingesetzt werden. Beispielsweise eignen sie sich als Solubilisatoren für kosmetische Öle. Sie besitzen ein gutes Solubilisiervermögen für Fette und Öle, wie Erdnußöl, Jojobaöl, Kokosnußöl, Mandelöl, Olivenöl, Palmöl, Ricinusöl, Sojaöl oder Weizenkeimöl oder für etherische Öle wie Latschenkieferöl, Lavendelöl, Rosmarinöl, Fichtennadelöl, Kiefernnadelöl, Eukalyptusöl, Pfefferminzöl, Salbeiöl, Bergamottöl, Terpentinöl, Melissenöl, Salbeiöl, Wacholderöl, Zitronenöl, Anisöl, Kardamonöl; Pfefferminzöl, Campheröl etc. oder für Mischungen aus diesen Ölen.

Weiterhin können die erfindungsgemäß verwendeten Polymere als Solubilisatoren für in Wasser schwerlösliche oder unlösliche UV-Absorber wie beispielsweise 2-Hydroxy-4-methoxybenzophenon (Uvinul^{®} M 40, Fa. BASF), 2,2',4,4'-Tetrahydroxybenzophenon (Uvinul^{®} D 50), 2,2'-Dihydroxy-4,4'-dimethoxybenzophenon (Uvinul^{®} D49), 2,4-Dihydroxybenzophenon (Uvinul^{®} 400), 2-Cyano-3,3-diphenylacrylsäure-2'-ethylhexylester (Uvinul^{®} N 539), 2,4,6-Trianilino-p-(carbo-2'-ethylhexyl-1'-oxi)-1,3,5-triazin (Uvinul® T 150), 3-(4-Methoxybenzyliden)-campher (Eusolex^{®} 6300, Fa. Merck), N,N-Dimethyl-4-aminobenzoesäure-2-ethylhexylester (Eusolex^{®} 6007), Salicylsäure-3,3,5-trimethylcyclohexylester, 4-Isopropyldibenzoylmethan (Eusolex^{®} 8020), p-Methoxyzimtsäure-2-ethylhexylester und p-Methoxyzimtsäure-2-isoamylester sowie Mischungen davon verwendet werden.

Gegenstand der vorliegenden Erfindung sind daher auch kosmetische Zubereitungen, die mindestens einen der erfindungsgemäß verwendeten Pfropfpolymerisate der eingangs genannten Zusammensetzung als Solubilisatoren enthalten. Bevorzugt sind solche Zubereitungen, die neben dem Solubilisator einen oder mehrere schwerlösliche kosmetische Wirkstoffe, beispielsweise die oben genannten Öle oder UV-Absorber enthalten.

Bei diesen Formulierungen handelt es sich um Solubilisate auf Wasser oder Wasser/Alkohol Basis. Die erfindungsgemäßen Solubilisatoren werden im Verhältnis von 0,2:1 bis 20:1, bevorzugt 1:1 bis 15:1, besonders bevorzugt 2:1 bis 12:1 zum schwerlöslichen kosmetischen Wirkstoff eingesetzt.

Der Gehalt an erfindungsgemäß verwendetem Solubilisator in der kosmetischen Zubereitung liegt, abhängig vom Wirkstoff, im Bereich von 1 bis 50 Gew.-%, bevorzugt 3 bis 40 Gew.-%, besonders bevorzugt 5 bis 30 Gew.-%.

Zusätzlich können dieser Formulierung weitere Hilfsstoffe zugesetzt werden, beispielsweise nichtionische, kationische oder anionische Tenside wie Alkylpolyglycoside, Fettalkoholsulfate, Fettalkoholethersulfate, Alkansulfonate, Fettalkoholethoxilate, Fettalkoholphosphate, Alkylbetaine, Sorbitanester, POE-Sorbitanester, Zuckerfettsäureester, Fettsäurepolyglycerinester, Fettsäurepartialglyceride, Fettsäurecarboxylate, Fettalkoholsulfosuccinate, Fettsäuresarcosinate, Fettsäureisethionate, Fettsäuretaurinate, Zitronensäureester, Silikon-Copolymere, Fettsäurepolyglykolester, Fettsäureamide, Fettsäurealkanolamide, quartäre Ammoniumverbindungen, Alkylphenoloxethylate, Fettaminoxethylate, Cosolventien wie Ethylenglykol, Propylenglykol, Glycerin u.a..

Als weitere Bestandteile können natürliche oder synthetische Verbindungen, z.B. Lanolinderivate, Cholesterinderivate, Isopropylmyristat, Isopropylpalmitat, Elektrolyte, Farbstoffe, Konservierungsmittel, Säuren (z.B. Milchsäure, Zitronensäure) zugesetzt werden.

Diese Formulierungen finden beispielsweise in Badezusatzpräparaten wie Badeölen, Rasierwässern, Gesichtswässern, Mundwässern, Haarwässern, Eau de Cologne, Eau de Toilette sowie in Sonnenschutzmitteln Verwendung.

### Beschreibung der Solubilisierungsmethode:

Bei der Herstellung der Solubilisate für kosmetische Formulierungen können die erfindungsgemäß verwendeten Pfropfpolymerisate als 100%ige Substanz oder bevorzugt als wäßrige Lösung eingesetzt werden.

Üblicherweise wird der zu verwendende schwerlösliche kosmetische Wirkstoff in einer Schmelze des Solubilisators gelöst und anschließend unter ständigem Rühren mit demineralisiertem Wasser versetzt.

Es kann aber auch der Solubilisator in Wasser gelöst und mit dem jeweils zu verwendenden schwerlöslichen kosmetischen Wirkstoff intensiv vermischt werden.

### Solubilisatoren für pharmazeutische Anwendungen:

Die erfindungsgemäß verwendeten Pfropfpolymerisate eignen sich ebenso für die Verwendung als Solubilisator in pharmazeutischen Zubereitungen jeder Art, die dadurch gekennzeichnet sind, daß sie einen oder mehrere in Wasser schwer lösliche oder wasserunlösliche Arzneistoffe sowie Vitamine und/oder Carotinoide enthalten können. Insbesondere handelt es sich dabei um wäßrige Lösungen bzw. Solubilisate zur oralen oder besonders bevorzugt zur parenteralen Applikation, wie z.B. Injektionslösungen zur intravenösen, intramuskulären oder subkutanen oder intraperitonealen Applikation.

Desweiteren eignen sich die Pfropfpolymerisate zum Einsatz in oralen Darreichungsformen wie Tabletten, Kapseln, Pulvern, Lösungen. Hier können Sie den schwerlöslichen Arzneistoff mit einer erhöhten Bioverfügbarkeit zur Verfügung stellen.

Bei der parenteralen Applikation können neben Solubilisaten auch Emulsionen, beispielsweise Fettemulsionen eingesetzt werden. Auch für diesen Zweck eignen sich die erfindungsgemäß verwendeten Pfropfpolymerisate um einen schwerlöslichen Arzneistoff zu verarbeiten.

Pharmazeutische Formulierungen der oben genannten Art können durch Verarbeiten der Pfropfpolymerisate mit pharmazeutischen Wirkstoffen nach herkömmlichen Methoden und unter Einsatz bekannter und neuer Wirkstoffe erhalten werden.

Die erfindungsgemäße Anwendung kann zusätzlich pharmazeutische Hilfsstoffe und/oder Verdünnungsmittel enthalten. Als Hilfsstoffe werden Cosolventien, Antioxidantien, Konservierungsmittel besonders aufgeführt.

Die verwendeten pharmazeutischen Wirkstoffe sind in Wasser unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Als Beispiele seien hier Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Die Verwendung der Pfropfpolymerisate als Lösungsvermittler kann beispielsweise in der Weise erfolgen, daß der Wirkstoff in dem Solubilisator, gegebenenfalls unter Erwärmen, dispergiert oder gelöst wird und unter Rühren mit Wasser vermischt wird.

Eine andere Herstellvariante ist das Auflösen des Solubilisators in der wäßrigen Phase, gegebenenfalls unter leichtem Erwärmen und das anschließende Lösen des Wirkstoffs in der wäßrigen Solubilisatorlösung. Das gleichzeitige Auflösen von Solubilisator und Wirkstoff in der wäßrigen Phase ist ebenfalls möglich.

Es ist auch möglich, den Solubilisator zusammen mit dem Wirkstoff in einem organischen Lösungsmittel, beispielsweise Ethanol, Isopropanol oder Aceton, gegebenenfalls unter Erwärmen zu lösen und diese Lösung mit ebenfalls erwärmtem Wasser vorsichtig zu vermischen. Nach Abdestillieren des organischen Lösungsmittels erhält man ein klares bzw. opaleszentes Wirkstoffsolubilisat.

Gegenstand der Erfindung sind auch pharmazeutische Zubereitungen, die mindestens einen der erfindungsgemäß verwendeten Pfropfpolymerisate als Solubilisator enthalten. Bevorzugt sind solche Zubereitungen, die neben dem Solubilisator einen in Wasser schwerlöslichen oder wasserunlöslichen pharmazeutischen Wirkstoff, beispielsweise aus den oben genannten Indikationsgebieten enthalten.

Besonders bevorzugt sind von den oben genannten pharmazeutischen Zubereitungen solche, bei denen es sich um parenteral applizierbare Formulierungen handelt.

Der Gehalt an erfindungsgemäß verwendetem Solubilisator in der pharmazeutischen Zubereitung liegt, abhängig vom Wirkstoff, im Bereich von 1 bis 50 Gew.-%, bevorzugt 3 bis 40 Gew.-%, besonders bevorzugt 5 bis 30 Gew.-%.

### Solubilisatoren für Lebensmittelzubereitungen:

Neben der Anwendung in der Kosmetik und Pharmazie eignen sich die erfindungsgemäß verwendeten Pfropfpolymerisaten auch als Solubilisatoren im Lebensmittelbereich für schwer wasserlösliche oder wasserunlösliche Nähr-, Hilfs- oder Zusatzstoffe, wie z.B. fettlösliche Vitamine oder Carotinoide. Als Beispiele seien klare, mit Carotinoiden gefärbte Getränke genannt.

### Solubilisatoren für Pflanzenschutzzubereitungen:

Die Anwendung der Pfropfpolymerisate als Solubilisatoren in der Agrochemie kann u.a. Formulierungen umfassen, die Pestizide, Herbizide, Fungizide oder Insektizide enthalten, vor allem auch solche Zubereitungen von Pflanzenschutzmitteln, die als Spritz- oder Gießbrühen zum Einsatz kommen.

In den folgenden Beispielen wird die Herstellung und Verwendung der erfindungsgemäßen Pfropfpolymerisate näher erläutert.

### Beispiele 1 - 3

### Pfropfpolymerisation von Vinylacetat mit Polyethylenglykol

Die Pfropfpolymerisate wurden nach der DE-PS 10 77 430 in der Weise hergestellt, daß man auf das jeweils in Tabelle 1 angegebene Polyethylenoxid (30 Gew.-Teile) 70 Gew.-Teile Vinylacetat bei 105°C unter Verwendung von 2,25 Gew.-%, bezogen auf die bei der Pfropfcopolymerisation eingesetzten Monomeren an Dibenzoylperoxid aufpolymerisierte.

**Tabelle 1:**

| Beispiel | Gew.-Teile Vinylacetat | Mw Polyethylenoxid | K-Wert (1% in Aceton) | Molekulargewicht (Mw) |
|---|---|---|---|---|
| 1 | 70 | 6000 | 21 | 20000 |
| 2 | 70 | 4000 | 21 | 20000 |
| 3 | 70 | 1500 | 15 | 10000 |

### Kosmetische Formulierungen

### Beispiel 4

6 g Solubilisator, hergestellt nach den Beispielen 1 und 2 wurden mit jeweils 1 g der in Tabelle 2 aufgeführten etherischen Öle bzw. Parfümöle mittels Magnetrührer innig gemischt. Unter ständigem Rühren wurde mit einer Bürette langsam demineralisiertes Wasser ad 100 g hinzugefügt. Die erhaltenen Formulierungen besaßen folgende Zusammensetzung:
1 Gew.-% etherisches oder kosmetisches Öl,
6 Gew.-% Solubilisator,
93 Gew.-% Wasser

**Tabelle 2:**

| | | |
|---|---|---|
| Solubilisator | etherisches Öl | Aussehen der Formulierung |
| | | |
| Beispiel 1 | Fichtennadelöl | opales Solubilisat |
| Beispiel 1 | Rosmarinöl | opales Solubilisat |
| Beispiel 1 | Lavendelöl | klares Solubilisat |
| Beispiel 1 | After Shave "Minos" der Fa. Drom | opales Solubilisat |
| Beispiel 2 | Latschenkieferöl | klares Solubilisat |
| Beispiel 2 | Lavendelöl | opales Solubilisat |

### Beispiel 5

### Sonnenschutzmittel

25 g Polymer gemäß Beispiel 2 wurden bei ca. 60 °C geschmolzen und 2,5 g Uvinul^{®} T 150 (Fa. BASF) wurden in der Schmelze gelöst. Anschließend wurde eine auf 60°C erwärmte Mischung von 62,5 g Aqua bidest und 10 g Glycerol vorsichtig unter Rühren hinzugetropft. Es entstand eine klare Lösung, die nach Erkalten auf Raumtemperatur in ein geeignetes Behältnis abgefüllt wurde.

### Pharmazeutische Formulierungen

### Beispiel 6

### Diazepam Injektionslösung

400 mg Polymer gemäß Beispiel 2 wurden in 1578 mg Aqua bidest gelöst. Anschließend wurden 10 mg Diazepam zu der Solubilisatorlösung gegeben und gerührt bis der Arzneistoff gelöst war. Die Lösung wurde mit 2 mg Natriumdisulfit und 10 mg Benzylalkohol konserviert und nach üblichen Methoden sterilfiltriert und in Injektionsfläschen gefüllt.

### Beispiel 7

### 17-β-Estradiol Gelatine Kapseln

100 mg 17-β-Estradiol wurden mit 10 g Polymer gemäß Beispiel 2, 80 g geschmolzenem PEG 6000 sowie 10 g Ethanol gemischt und anschließend direkt in flüssiger Form in Kapseln gefüllt.

### Beispiel 8

### Orale Ciclosporin Formulierung (flüssig befüllte Kapsel)

100 g Ciclosporin A wurden in 770 g Polymer gemäß Beispiel 2, 100 ml Ethanol und 75 ml Propylengykol gelöst und die viskose klare Lösung wurde anschließend in Kapseln abgefüllt. Diese Lösung war unbegrenzt mit Wasser verdünnbar.

### Beispiel 9

### Diazepam Emulsion zur parenteralen Applikation

160 g Polymer gemäß Beispiel 2 wurden in 660 g Aqua bidest gelöst. 10 g Diazepam wurden in einer 1:1 Mischung aus Sojaöl und Miglyol Öl (Ölphase betrug 200 g) dispergiert oder gelöst. Zusätzlich wurden 10 g Sojalecithin eingesetzt, das in der Ölphase gelöst wurde. Die beiden Phasen wurden vordispergiert und anschließend per Hochdruckhomogenisation emulgiert.

### Beispiel 10

### 17-β-Estradiol Tablette

10 g 17-β-Estradiol wurden mit 50 g Polymer gemäß Beispiel 2 geschmolzen. Die Schmelze wurde auf 940 g Ludipress^{®} (Fa. BASF) gezogen. Das Granulat wurde anschließend mit 0,5 g Mg-stearat gemischt und die Mischung anschließend tablettiert.

### Beispiel 11

### Diazepam-haltiges Pulver

Diazepam und das Polymer gemäß Beispiel 2 als Solubilisator wurden in einem organischen Lösungsmittel z. B. Ethanol gelöst. Anschließend wird ein Trägerstoff, z. B. Sorbitol hinzugefügt und ebenfalls gelöst. Anschließend wird das Lösungsmittel entfernt und die Mischung im Vakuum getrocknet.

### Beispiel 12

### Solubilisierende Wirkung am Beispiel 17-β-Estradiol, Nifedipin und Clotrimazol

Zu einer Schmelze der erfindungsgemäß verwendeten Polymere wurden bei 65°C jeweils 17-β-Estradiol, Nifedipin und Clotrimazol im Überschuß zugegeben. Anschließend wurde die wäßrige Phase zugesetzt und die Mischung bis zur Einstellung des Gleichgewichts langsam gerührt. Der überschüssige Feststoff wurde abfiltriert und der Gehalt an gelöstem Wirkstoff im Filtrat bestimmt (siehe Tabelle 3).

**Tabelle 3:**

| Copolymer | Solubilisierung [Gew.-%] | | |
|---|---|---|---|
| | | | |
| | 17-β-Estradiol | Nifedipin | Clotrimazol |
| | | | |
| Polymer (Beispiel 1) | 0,12 | 0,41 | 0,28 |
| Polymer (Beispiel 2) | 0,11 | 0,43 | 0,29 |
| Polymer (Beispiel 3) | 0,15 | 0,39 | - |
| | | | |
| Vergleich: | | | |
| | | | |
| Phosphatpuffer, pH 7,0 | 0,0 | | 0,0 |
| Tween^{®} 80 | 0,09 | 0,28 | 0,03 |
| Cremophor^{®} EL | 0,06 | 0,27 | 0,01 |

### Beispiel 13

### Bestimmung der Hämolyseaktivität im RBC-Test

In einem RBC-Test (Red Blood Cell) an Kaninchenerythrozyten wurde die hämolytische Aktivität der beanspruchten Verbindungen getestet (siehe Tabelle 4). Die Inkubationszeit betrug 60 min bei Raumtemperatur.

**Tabelle 4:**

| Verbindung | Hämolyse der 1 %-igen Lösungen in Phopsphatpuffer |
|---|---|
| Phosphatpuffer pH 7,0 | keine |
| Sorbitanfettsäureester (Tween^{®} 80) | keine |
| Ethoxyliertes Ricinusöl (Cremophor^{®} EL) | keine |
| Beispiel 1 | keine |
| Beispiel 2 | keine |
| Beispiel 3 | keine |

### Beispiel 14

Verträglichkeit am Hund

Nach intravenöser Injektion einer 5 %-igen wäßrigen Lösung der beanspruchten Verbindungen am Hund wurde die Bluthistaminauschüttung verfolgt (Tabelle 5).

**Tabelle 5:**

| Verbindung | 5 min. vor Applikation | 5 min. nach Applikation | 15 min. nach Applikation |
|---|---|---|---|
| Sorbitanfettsäureester (Tween^{®} 80) | 3*⁾ | 14142 | 58065 |
| Solutol^{®} HS 15 | 5 | 138 | 220 |
| Polymer aus Beispiel 2 | 5 | 312 | 104 |

| | | | |
|---|---|---|---|
| *⁾ Die Zahlenangaben stellen Bluthistaminspiegel in ng/ml dar. | | | |

## Patentansprüche

1. Verwendung von Pfropfpolymerisaten, die erhältlich sind durch Pfropfen von
a) Polyalkylenoxiden mit
b) mindestens einem Monomeren, ausgewählt aus der Gruppe
b₁) C₁-C₃₀-Alkylester von monoethylenisch ungesättigten C₃-C₈-Carbonsäuren;
b₂) Vinylester von aliphatischen C₁-C₃₀-Carbonsäuren;
b₃) C₁-C₃₀-Alkyl-Vinylether;
b₄) N-C₁-C₃₀-Alkyl-substituierter Amide von monoethylenisch ungesättigten C₃-C₈-Carbonsäuren;
b₅) N,N-C₁-C₃₀-Dialkyl-substituierter Amide von monoethylenisch ungesättigten C₃-C₈-Carbonsäuren
als Solubilisator.

2. Verwendung von Pfropfpolymerisaten nach Anspruch 1, **dadurch gekennzeichnet, daß** sie erhältlich sind durch Pfropfen von
a) Polyalkylenoxiden eines mittleren Molekulargewichtes von 300 bis 100.000 (nach dem Zahlenmittel) auf Basis von Ethylenoxid, Propylenoxid und/oder Butylenoxid mit
b) mindestens einem Monomeren, ausgewählt aus der Gruppe
b₁) C₁-C₃₀-Alkylester von monoethylenisch ungesättigten C₃-C₈-Carbonsäuren;
b₂) Vinylester von aliphatischen C₁-C₃₀-Carbonsäuren;
b₃) C₁-C₃₀-Alkyl-Vinylether;
b₄) N-C₁-C₃₀-Alkyl-substituierter Amide von monoethylenisch ungesättigten C₃-C₈-Carbonsäuren ;
b₅) N,N-C₁-C₃₀-Dialkyl-substituierter Amide von monoethylehisch ungesättigten C₃-C₈-Carbonisäuren.

3. Verwendung von Pfropfpolymerisaten nach Anspruch 1, **dadurch gekennzeichnet, daß** sie erhältlich sind durch Pfropfen von
a) Polyalkylenoxiden eines mittleren Molekulargewichtes (nach dem Zahlenmittel) von 500 bis 20.000 auf Basis von Ethylenoxid, und/oder Propylenoxid mit
b) mindestens einem Monomeren, ausgewählt aus der Gruppe
b₁) C₁-C₁₂-Alkylester von monoethylenisch ungesättigten C₃-C₈-Carbonsäuren ;
b₂) Vinylester von aliphatischen C₁-C₁₂-Carbonsäuren ;
b₃) C₁-C₁₂-Alkyl-Vinylether ;
b₄) N-C₁-C₁₂-Alkyl-substituierter Amide von monoethylenisch ungesättigten C₃-C₈-Carbonsäuren;
b₅) N,N-C₁-C₁₂-Dialkyl-substituierter Amide von monoethylenisch ungesättigten C₃-C₈-Carbonsäuren.

4. Verwendung von Pfropfpolymerisaten nach den Ansprüchen 1 bis 3 mit einem mittleren Molekulargewicht von 1000 bis 30000 g/mol.

5. Verwendung von Pfropfpolymerisaten nach den Ansprüchen 1 bis 4 als Solubilisatoren in pharmazeutischen und kosmetischen Zubereitungen.

6. Verwendung von Pfropfpolymerisaten nach den Ansprüchen 1 bis 4 als Solubilisatoren in Lebensmittelzubereitungen.

## Claims

1. The use of graft copolymers obtainable by grafting
a) polyalkylene oxides with
b) at least one monomer selected from the group of
b₁) C₁-C₃₀-alkyl esters of monoethylenically unsaturated C₃-C₈-carboxylic acids;
b₂) vinyl esters of aliphatic C₁-C₃₀-carboxylic acids;
b₃) C₁-C₃₀-alkyl vinyl ethers;
b₄) N-C₁-C₃₀-alkyl-substituted amides of monoethylenically unsaturated C₃-C₈-carboxylic acids;
b₅) N,N-C₁-C₃₀-dialkyl-substituted amides of monoethylenically unsaturated C₃-C₈-carboxylic acids
as solubilizer.

2. The use of graft copolymers according to claim 1, wherein they are obtainable by grafting
a) polyalkylene oxides with a number average molecular weight of from 300 to 100,000 and based on ethylene oxide, propylene oxide and/or butylene oxide with
b) at least one monomer selected from the group of
b₁) C₁-C₃₀-alkyl esters of monoethylenically unsaturated C₃-C₈-carboxylic acids;
b₂) vinyl esters of aliphatic C₁-C₃₀-carboxylic acids;
b₃) C₁-C₃₀-alkyl vinyl ethers;
b₄) N-C₁-C₃₀-alkyl-substituted amides of monoethylenically unsaturated C₃-C₈-carboxylic acids;
b₅) N,N-C₁-C₃₀-dialkyl-substituted amides of monoethylenically unsaturated C₃-C₈-carboxylic acids.

3. The use of graft copolymers according to claim 1, wherein they are obtainable by grafting
a) polyalkylene oxides with a number average molecular weight of from 500 to 20,000 and based on ethylene oxide and/or propylene oxide with
b) at least one monomer selected from the group of
b₁) C₁-C₁₂-alkyl esters of monoethylenically unsaturated C₃-C₈-carboxylic acids;
b₂) vinyl esters of aliphatic C₁-C₁₂-carboxylic acids;
b₃) C₁-C₁₂-alkyl vinyl ethers;
b₄) N-C₁-C₁₂-alkyl-substituted amides of monoethylenically unsaturated C₃-C₈-carboxylic acids;
b₅) N,N-C₁-C₁₂-dialkyl-substituted amides of monoethylenically unsaturated C₃-C₈-carboxylic acids.

4. The use of graft copolymers according to claims 1 to 3 having an average molecular weight of from 1000 to 30,000 g/mol.

5. The use of graft copolymers according to claims 1 to 4 as solubilizers in pharmaceutical and cosmetic preparations.

6. The use of graft copolymers according to claims 1 to 4 as solubilizers in food products.

## Revendications

1. Utilisation de polymères greffés qu'on obtient par greffage de :
a) oxydes de polyalkylène avec
b) au moins un monomère sélectionné parmi le groupe des :
b₁) alkylesters en C₁-C₃₀ d'acides carboxyliques en C₃-C₈ monoéthyléniquement insaturés;
b₂) vinylesters d'acides carboxyliques aliphatiques en C₁-C₃₀;
b₃) alkyle en C₁-C₃₀-vinyléthers ;
b₄) amides d'acides carboxyliques en C₃-C₈ monoéthyléniquement insaturés substitués par un N-alkyle en C₁-C₃₀;
b₅) amides d'acides carboxyliques en C₃-C₈ monoéthyléniquement insaturés substitués par un N,N-dialkyle en C₁-C₃₀
comme agent de solubilisation.

2. Utilisation de polymères greffés selon la revendication 1, **caractérisée en ce qu'**on les obtient par greffage de :
a) oxydes de polyalkylène d'un poids moléculaire moyen de 300 à 100 000 (selon la moyenne en nombre) à base d'oxyde d'éthylène, d'oxyde de propylène et/ou d'oxyde de butylène avec
b) au moins un monomère sélectionné parmi le groupe des :
b₁) alkylesters en C₁-C₃₀ d'acides carboxyliques en C₃-C₈ monoéthyléniquement insaturés;
b₂) vinylesters d'acides carboxyliques aliphatiques en C₁-C₃₀ ;
b₃) alkyle en C₁-C₃₀-vinyléthers ;
b₄) amides d'acides carboxyliques en C₃-C₈ monoéthyléniquement insaturés substitués par un N-alkyle en C₁-C₃₀;
b₅) amides d'acides carboxyliques en C₃-C₈ monoéthyléniquement insaturés substitués par un N,N-dialkyle en C₁-C₃₀.

3. Utilisation de polymères greffés selon la revendication 1, **caractérisée en ce qu'**on les obtient par greffage de :
a) oxydes de polyalkylène d'un poids moléculaire moyen (selon la moyenne en nombre) de 500 à 20 000 à base d'oxyde d'éthylène et/ou d'oxyde de propylène avec
b) au moins un monomère sélectionné parmi le groupe des :
b₁) alkylesters en C₁-C₁₂ d'acides carboxyliques en C₃-C₈ monoéthyléniquement insaturés;
b₂) vinylesters d'acides carboxyliques aliphatiques en C₁-C₁₂;
b₃) alkyle en C₁-C₁₂-vinyléthers;
b₄) amides d'acides carboxyliques en C₃-C₈ monoéthyléniquement insaturés substitués par un N-alkyle en C₁-C₁₂ ;
b₅) amides d'acides carboxyliques en C₃-C₈ monoéthyléniquement insaturés substitués par un N,N-dialkyle en C₁-C₁₂.

4. Utilisation de polymères greffés selon les revendications 1 à 3 avec un poids moléculaire moyen de 1000 à 30 000 g/mole.

5. Utilisation de polymères greffés selon les revendications 1 à 4 comme agents de solubilisation dans des préparations pharmaceutiques et cosmétiques.

6. Utilisation de polymères greffés selon les revendications 1 à 4 comme agents de solubilisation dans des préparations de produits alimentaires.
